Europäisches Patentamt

European Patent Office

Office européen des brevets

(19)

(11) Numéro de publication: **0 272 989 B1**

# FASCICULE DE BREVET EUROPEEN

(45) Date de publication de fascicule du brevet:
**16.10.91**

(51) Int. Cl.⁵: **A61F 13/08**

(21) Numéro de dépôt: **87420311.0**

(22) Date de dépôt: **18.11.87**

(54) **Procédé de blocage local de dispositifs de contention élastiques.**

(30) Priorité: **19.11.86 FR 8616512**

(43) Date de publication de la demande:
**29.06.88 Bulletin 88/26**

(45) Mention de la délivrance du brevet:
**16.10.91 Bulletin 91/42**

(84) Etats contractants désignés:
**BE CH DE ES GB IT LI**

(56) Documents cités:
**US-A- 4 084 584**
**US-A- 4 287 885**

(73) Titulaire: **RICHARD FRERES S.A.**

**F-42530 Saint Genest Lerpt (Loire)(FR)**

(72) Inventeur: **Richard, Dominique**
**3 rue des Verchères**
**F-42530 Saint Genest Lerpt(FR)**

(74) Mandataire: **Maureau, Philippe et al**
**Cabinet Germain & Maureau Le Britannia -**
**Tour C 20, bld Eugène Déruelle Boîte Posta-**
**le 3011**
**F-69392 Lyon Cédex 03(FR)**

## Description

La présente invention concerne un procédé de blocage local de dispositifs de contention élastiques, spécialement prévu pour limiter localement l'extensibilité de l'article et augmenter simultanément la contention.

Le procédé selon l'invention est bien adapté, sans qu'il en résulte pour autant une limitation à sa portée, aux dispositifs de contention des articulations telles que phalanges, vertèbres et est spécialement intéressant pour les dispositifs de contention d'articulations comportant un élément saillant (cheville, genou, coude, etc...).

Le procédé de blocage local selon l'invention est caractérisé en ce qu'il consiste à sou mettre une zone localisée de l'une des deux faces d'un tissu ou tricot possédant des caractéristiques élastiques à l'action de la chaleur a un degré tel que lesdites caractéristiques élastiques soient partiellement ou totalement supprimées sur ladite zone localisée, tout en restant essentiellement maintenues sur l'autre face.

L'action de suppression locale des caractéristiques élastiques peut également s'accompagner d'une certaine rigidification contribuant, par ailleurs, à renforcer localement l'effet de contention ; ceci est, bien entendu, fonction tant de la nature de la matière constitutive de la zone où est exercée l'action de la chaleur, que du taux de chaleur appliquée.

D'autre part, et ceci constitue également un avantage important de l'invention, on peut, grâce à ce procédé de blocage local appliqué dans le cas de tricots, réaliser par découpe dans ladite zone ainsi traitée des ouvertures nettes, sans qu'il se produise de "défilement" des fils ; du fait du blocage de l'élasticité préalablement mentionné, il est possible de communiquer dès le départ auxdites ouvertures la forme qu'elles doivent présenter lors de l'application du dispositif de contention.

Il faut rappeler que, dans le cas où il est nécessaire que le dispositif de contention présente une ouverture circulaire, destinée par exemple à recevoir la rotule du coude ou du talon, on ne pouvait, dans les dispositifs de la technique antérieure, du fait de leur élasticité, réaliser ces ouvertures que sous forme ovale, l'aspect circulaire n'étant communiqué à l'ouverture qu'une fois l'article mis en place sur le porteur, ou bloquer le rond par d'autres artifices (rajout de divers matériaux rigides par confection notamment, comme cela est le cas dans le brevet US N° 4 287 885) qui créent des surépaisseurs. Grâce au procédé selon l'invention, on peut, dès la confection de l'article, réaliser des ouvertures parfaitement circulaires et le demeurant ultérieurement, malgré l'extensibilité du reste de l'article.

La présente invention sera mieux comprise et ses avantages ressortiront bien de la description qui suit qui l'illustre, sans toutefois la limiter, en référence au dessin schématique annexé dans lequel :

Figure 1 est une vue en coupe schématique d'un tricot d'une certaine épaisseur avant application du procédé selon l'invention ;

Figure 2 est une vue similaire à figure 1, après application du procédé selon l'invention sur une zone localisée de tricot;

Figure 3 est une vue de face, en perspective, d'une genouillère réalisée avec un tricot auquel a été appliqué le procédé selon l'invention.

Sur les figures, le tissu ou tricot est représenté par 2 et la zone localisée sur laquelle est appliqué le traitement thermique par 3.

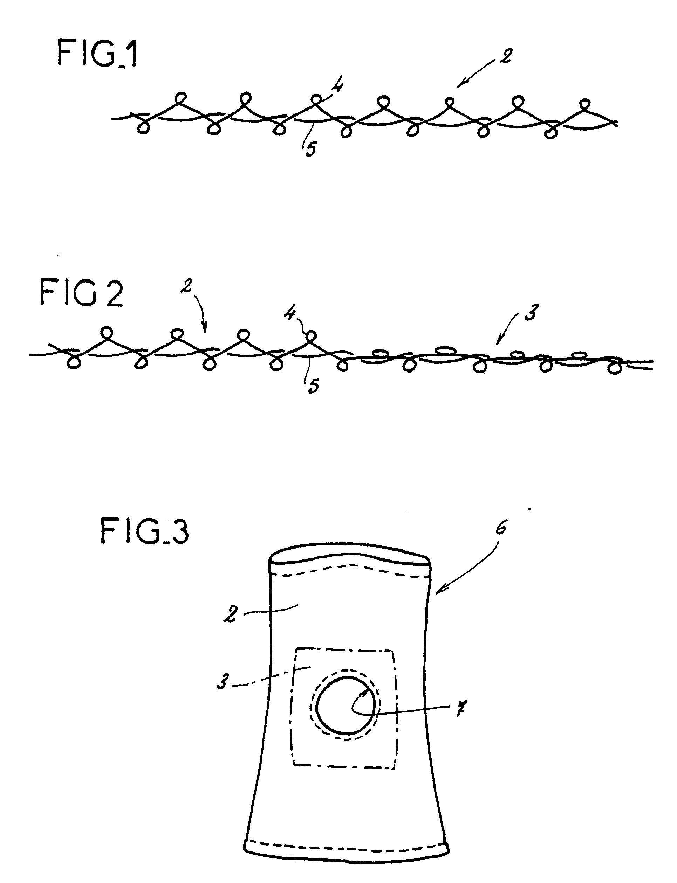

Dans le mode de représentation décrit aux figures 1 et 2, le tricot 2 est essentiellement réalisé en fils ou fibres synthétiques 4 et un fil de trame élastique 5 est inséré entre les mailles dudit tricot.

Selon l'invention, on exerce localement, sur une zone prédéterminée 3 du tricot 2 et sur une seule face de celui-ci une action thermique, par exemple par apposition, avec ou sans pression, d'une plaque métallique convenablement chauffée ; cette action thermique peut être également exercée par convection au travers d'un cadre, découpé à la dimension de ladite zone et exerçant un effet protecteur sur les parties voisines.

Cette application, éventuellement faite sous une certaine pression, occasionne un retrait de la partie des fils synthétiques 4 se trouvant sur cette face (figure 2) et ce retrait provoque un blocage, au moins partiel, de la trame élastique 5 ; ce blocage ne prend place que sur la zone 3 soumise à l'action thermique et non pas sur les zones environnantes ni sur la face opposée du tricot ; le reste du tricot 2 conserve donc toutes ses propriétés élastiques.

Le taux de chaleur -éventuellement appliquée sous pression- sera, bien évidemment, fonction de la nature du fil synthétique formant les mailles du tricot, et l'homme du métier, connaissant les propriétés de retrait thermique dudit fil, sera à même de la déterminer.

Dans certains cas, et selon les exigences à satisfaire, ce retrait pourra s'accompagner d'une rigidification par exemple quand ou utilise des chlorofibres.

Ce procédé pourra être utilisé aussi avec des fibres naturelles (laine etc ...). On pourra agir aussi de manière à bloquer l'élastique lui-même, notamment grâce à son guipage ou à sa couverture. On peut favoriser l'effet en insérant des petits fils particulièrement sensibles à la chaleur. De plus, en ajoutant de la pression, voire de l'écrasement de chaleur, et ce sur une face par exemple, on favorise l'effet désiré, tout en conservant à l'autre face son aspect primaire, qui pouvait être un toucher doux, par exemple, De même, dans le cas d'un tricot double face, une face en fil synthétique et une face en fil naturel, le procédé selon l'invention sera appliqué sur la face en fil synthétique.

Sur le mode de réalisation représenté à la figure 3, le procédé selon l'invention a été appliqué sur une genouillère 6 et sur une zone 3 localement soumise au traitement thermique qui remplit alors deux fonctions : il permet, tout d'abord, la réalisation, sur la zone traitée, d'une ouverture circulaire 7 correspondant à l'échancrure rotulienne et qui demeure circulaire tout au long de la vie de l'article, malgré l'extensibilité générale de ce dernier. Le bord de l'ouverture 7 est bloquée et s'oppose à tout défilement des fils élastiques.

On réalise, d'autre part, autour de la rotule, un maintien différent de celui de l'ensemble de l'article de qui permet d'isoler la rotule de reste de la genouillère tout en ayant une ouverture qui plaque bien contre cette dite rotule.

Ce maintien peut être encore amélioré si l'on équipe ladite zone de dispositifs de blocage de l'articulation rotulaire et/ou de bandes de dérotation.

Comme on le voit, le procédé selon l'invention présente un intérêt tout particulier dans son application à la réalisation de dispositifs de maintien ou de contention des articulations.

Il est également applicable à tous les autres dispositifs de contention, dans lesquels il est souhaitable de disposer d'une ou plusieurs zones de formes variées selon l'effet désiré présentant une élasticité moindre et/ou une rigidité accrue : bas à varices, ceintures de maintien, etc...

Il est, bien entendu, que le procédé, décrit plus spécialement dans ses applications à un tricot, peut également s'appliquer à tout autre support textile ainsi qu'aux tricots et tissus doubles dont l'une des couches peut alors être, en certaines zones, différenciée de l'autre.

## Revendications

1. Procédé de blocage local de dispositifs de contention élastiques, caractérisé en ce qu'il consiste à soumettre une zone localisée (3) de l'une des deux faces d'un tissu ou tricot (2) possédant des caractéristiques élastiques à l'action de la chaleur à un degré tel que lesdites caractéristiques élastiques soient partiellement ou totalement supprimées sur ladite zone localisée, tout en restant essentiellement maintenues sur l'autre face.

2. Procédé selon la revendication 1, caractérisé en ce que le tissu ou tricot (2) est réalisé au moins partiellement à partir de fibres ou de fils synthétiques.

3. Procédé selon la revendication 1, caractérisé en ce que l'action de la chaleur est telle qu'une certaine rigidification accompagne la suppression partielle ou totale des caractéristiques élastiques.

## Claims

1. A process for the local locking of elastic containment appliances, *characterized in that* it consists in submitting a localized zone (3) of one of the two faces of a woven or knitted fabric (2) possessing elastic characteristics to the action of heat to such an extent that the said elastic characteristics are partly or wholly suppressed over the said localized zone, whilst being essentially maintained on the other face.

2. A process according to Claim 1, *characterized in that* the woven or knitted fabric (2) is produced, at least partly, from synthetic fibres or threads.

3. A process according to Claim 1, *characterized in that* the action of the heat is such that a certain rigidification accompanies the partial or total suppression of the elastic characteristics.

## Patentansprüche

1. Verfahren zur Herstellung eines nichtelastischen Teiles in einer elastischen orthopädischen Vorrichtung, dadurch gekennzeichnet, daß es darin besteht, einen lokalisierten Bereich (3) von der einen der beiden Seiten eines gewobenen oder gestrickten, elastische Eigenschaften besitzenden Stoffes (2) aus der Einwirkung von Hitze in einem Maße auszusetzen, daß die genannten elastischen Eigenschaften teilweise oder total im genannten lokalisierten Bereich verschwinden, wobei sie an der anderen Seite im wesentlichen erhalten bleiben.

2. Verfahren nach Anspruch 1, dadurch gekennzeichnet, daß der gewobene oder gestrickte Stoff (2) wenigstens teilweise aus Fasern oder Fäden aus Kunststoff hergestellt ist.

3. Verfahren nach Anspruch 1, dadurch gekennzeichnet, daß die Einwirkung von Hitze so erfolgt, daß das teilweise oder totale Verschwinden der elastischen Eigenschaften von einer gewissen Verstärkung begleitet ist.

FIG_1

FIG 2

FIG_3